# FASCICULE DE BREVET EUROPEEN

(11) **EP 1 017 328 B1**
(45) Date de publication et mention de la délivrance du brevet: **17.12.2003**
(21) Numéro de dépôt: 98944026.8
(22) Date de dépôt: 21.09.1998
(51) Int. Cl.: A61B 17/70

(54) **SYSTEME A VIS ET PLAQUE POUR L'OSTEOSYNTHESE DU RACHIS**
PLATTE- UND SCHRAUBENSYSTEM ZUR OSTEOSYNTHESE DER WIRBELSÄULE
SCREW AND PLATE SYSTEM FOR BACKBONE OSTEOSYNTHESIS

(30) Priorité: 23.09.1997 FR 9711811
(43) Date de publication de la demande: 12.07.2000
(73) Titulaire: Dimso Distribution Medicale du Sud Ouest, 33610 Cestas (FR)
(72) Inventeur: BACCELLI, Christian, F-33650 Saint Médard d'Eyrans (FR); CONCHY, Fréderic, F-33000 Bordeaux (FR); HENRY, Patrick, F-92300 Levallois Perret (FR)
(74) Mandataire: Le Forestier, Eric
(86) Numéro de dépôt international: FR9802010
(87) Numéro de publication internationale: WO99015094

(56) Documents cités:
- EP-A- 0 441 084
- EP-A- 0 727 188
- WO-A-95/19149

## Description

La présente invention a trait d'une façon générale aux systèmes d'ostéosynthèse du rachis, du type "plaque".

Un tel système est déjà connu et comporte au moins deux vis pédiculaires ancrées dans des vertèbres adjacentes à traiter, et une plaque d'union destinée à relier rigidement les têtes de vis les unes aux autres.

On connaît en particulier par EP-A-0 441 084 un tel système, dans lequel chaque vis pédiculaire possède une partie à section hexagonale pour la mise en place de la vis dans l'os, partie qui est surmontée par une tige filetée.

La plaque possède plusieurs ouvertures oblongues à travers lesquelles peuvent être engagées les différentes tiges filetées, et une rainure ouverte sur sa face inférieure pour bloquer la partie hexagonale de chaque vis pédiculaire contre la rotation, afin d'éviter son desserrage.

En association avec chaque vis pédiculaire est également prévu un étrier également traversé par la tige filetée de la vis, qui chevauche la plaque par le dessus, et un écrou est enfin serré sur la tige filetée pour emprisonner et bloquer, entre lui et la partie hexagonale de la vis, la plaque surmontée de son étrier.

Des reliefs complémentaires sont par ailleurs prévus sur la face supérieure de la plaque et sur la face inférieure de l'étrier pour éviter tout glissement longitudinal de la plaque par rapport à la vis.

Ce système de fixation, s'il donne généralement satisfaction, présente toutefois certains inconvénients.

Ainsi la nécessité de prévoir des reliefs destinés à éviter le glissement est dû au fait que la plaque et l'étrier coopèrent seulement par deux faces planes en compression, et leur absence risquerait d'entraîner des déplacements tout à fait indésirables entre les vertèbres.

Or ces reliefs, par l'usinage qu'ils nécessitent, accroissent sensiblement le coût de revient des plaques et des étriers.

En outre, ils peuvent gêner l'ajustement fin du positionnement du système, dans la mesure où ils ne permettent qu'un certain nombre de positions mutuelles discrètes entre la plaque et l'étrier, c'est-à-dire qu'un certain nombre de distances discrètes entre vis; en outre, lorsque l'écrou, avant serrage, est excessivement vissé, le coulissement mutuel de la plaque et de l'étrier lors des ajustements peut se trouver gêné.

Enfin ce système connu impose de rapporter par dessus la plaque, avant le vissage des écrous, les différents étriers, ce qui est une manoeuvre fastidieuse, qui induit également un risque de mauvais positionnement de ces étriers avant serrage.

La présente invention vise à pallier ces inconvénients et à proposer un système de liaison du genre plaque dans lequel l'usinage des différents composants soit beaucoup moins contraignant, tout en offrant une excellente stabilité.

Un autre objet de l'invention est de proposer un système dont la pose soit simplifiée, en évitant le montage fastidieux et instable d'étriers avant la pose des écrous.

Un autre objet encore de la présente invention est de proposer un système qui présente une certaine déformabilité élastique, afin de mieux répartir les contraintes entre les différentes vertèbres.

Ainsi la présente invention propose un système d'ostéosynthèse pour le rachis, comprenant au moins deux vis pédiculaires et une plaque d'union apte à relier les vis l'une à l'autre de façon essentiellement rigide, chaque vis possédant une partie filetée d'ancrage osseux et une tête à section non circulaire, ainsi qu'une tige filetée terminale apte à coopérer avec un écrou, la plaque possédant au moins une ouverture apte à être traversée par la tige filetée terminale d'une vis pour être emprisonnée entre ladite tête à section non circulaire et ledit écrou, et des moyens étant prévus pour réaliser un blocage angulaire entre la tête de chaque vis pédiculaire et la plaque, caractérisé en ce que les moyens de blocage angulaire comprennent un élément rapporté entre ladite plaque et ladite tête de la vis pédiculaire et traversé par la tige filetée terminale de cette dernière, cet élément réalisant une première coopération de formes bloquante avec ladite tête et une seconde coopération de formes bloquante avec ladite plaque, l'élément étant apte à réaliser la première coopération avec la zone de section non circulaire de la tête.

Des aspects préférés, mais non limitatifs, du système selon l'invention sont les suivants :
- la première coopération de formes bloquante est un chevauchement de ladite tête à section non circulaire par. ledit élément rapporté par le dessus.
- ledit élément rapporté comprend deux jambes latérales délimitant entre elles un canal à bords droits dont la largeur est légèrement supérieure à la distance entre deux pans parallèles de ladite tête de vis pédiculaire.
- la seconde coopération de formes bloquante est un chevauchement de ladite plaque par ledit élément rapporté par le dessous.
- ledit élément rapporté possède deux montants latéraux définissant entre eux un canal à bords relevés en oblique, et ladite plaque possède des chanfreins latéraux aptes à s'appuyer contre lesdits bords obliques.
- ladite plaque s'appuie sur ledit élément rapporté seulement au niveau desdits chanfreins.
- ledit élément rapporté comporte une âme en forme générale de disque traversée par une ouverture centrale pour le passage de la tige filetée terminale de la vis pédiculaire.
- la largeur de l'élément rapporté est sensiblement égale à celle de la plaque.
- le système comprend un jeu de vis pédiculaires et un jeu de plaques possédant différentes configurations de trous, avec un trou légèrement oblong de géométrie constante et un ou plusieurs trous allongés de longueurs différentes.
- l'écrou vient en appui directement contre la face supérieure de la plaque.

D'autres aspects, buts et avantages de la présente invention apparaîtront mieux à la lecture de la description détaillée suivante d'une forme de réalisation préférée de celle-ci, donnée à titre d'exemple et faite en référence aux dessins annexés, sur lesquels :
la figure 1 est une vue d'ensemble en perspective d'un implant rachidien selon l'invention,
la figure 2 est une vue en élévation de côté de l'implant,
la figure 3 est une autre vue en perspective de l'implant, à échelle agrandie,
la figure 4 est une vue en bout, en élévation, de l'implant,
la figure 5 est une vue en perspective d'une plaque utilisée dans l'implant,
la figure 6 est une vue en élévation de côté d'une autre version de la plaque,
la figure 7 est une vue en bout, en élévation, de la plaque de la figure 6,
la figure 8 est une vue de dessus de la plaque des figures 6 et 7,
les figures 9 à 11 sont des vues de dessus de trois autres versions de la plaque,
la figure 12 est une vue en élévation de face d'un élément de liaison et de blocage de l'implant,
la figure 13 est une vue de côté de l'élément de la figure 12, et
la figure 14 est une vue de dessus de l'élément des figures 12 et 13.

En référence aux dessins, on a représenté un implant pour l'ostéosynthèse du rachis du type "plaque", dont le principe consiste à ancrer une série de vis pédiculaires 10 dans des vertèbres adjacentes et à relier ces vis 10 entre elles de façon rigide à l'aide d'une plaque à orifices.

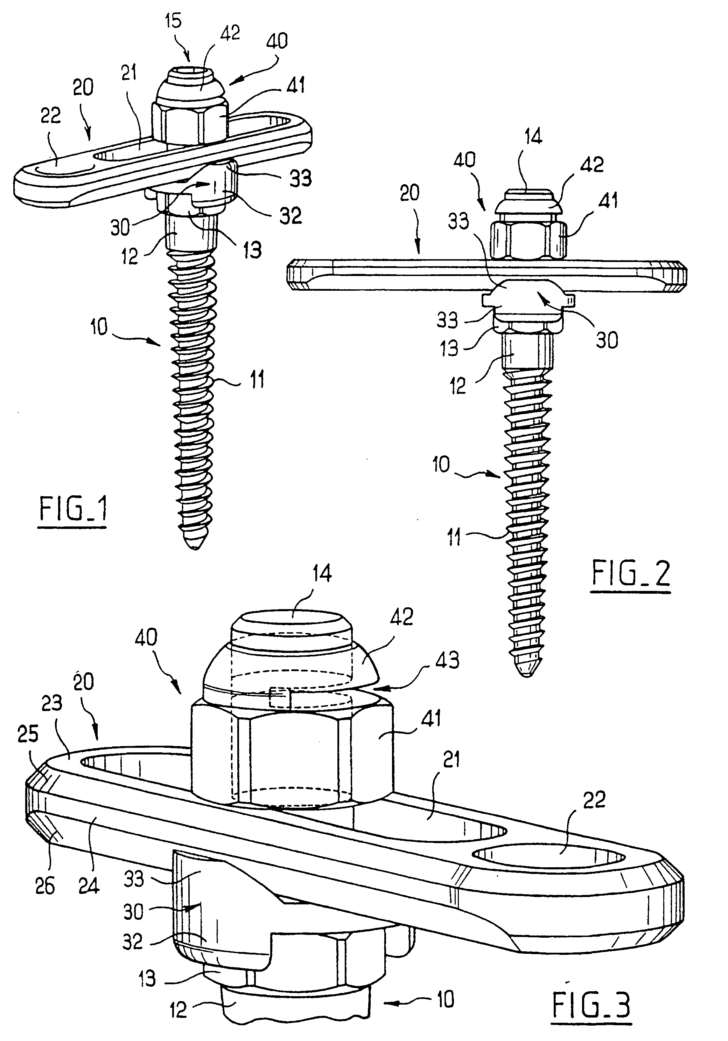

Les figures 1 à 4, par souci de clarté, montrent la coopération de la plaque avec une seule des vis.

Chaque vis 10 comprend, de façon classique en soi, d'une extrémité vers l'autre, une partie filetée 11 adaptée pour permettre son ancrage dans la vertèbre, un col 12, une tête 13 à section hexagonale pour permettre le vissage de la vis dans la vertèbre à l'aide d'une clé, et enfin une partie terminale cylindrique filetée 14, dont le filetage est de diamètre et de pas constants. Eventuellement, le sommet de la partie filetée 14 comporte une empreinte en creux à six pans 15 (voir figure 1) permettant, en variante, le vissage de la vis 10 dans la vertèbre par le dessus à l'aide d'une clé appropriée.

La plaque d'union, désignée par la référence 20, présente une forme généralement plane et arrondie, avec des extrémités semi-circulaires. Elle est délimitée par une face supérieure 23, une face inférieure 27, une face latérale 24, un premier chanfrein 25, à 45°, à la transition entre la face supérieure 23 et la face latérale 24, et un second chanfrein 26, également à 45°, à la transition entre la face inférieure 27 et la face latérale 24.

On observe ici que le chanfrein inférieur 26 est plus large au niveau des flancs de la plaque qu'au niveau de ses extrémités.

La plaque 20 est traversée dans le sens de son épaisseur par une série d'ouvertures destinées à son assemblage avec les vis pédiculaires, ces ouvertures étant orientées dans le sens longitudinal de la plaque. Il est prévu typiquement une ouverture oblongue 22 faible longueur, et une ou plusieurs ouvertures oblongues 21 de plus grande longueur, différents agencements de ces ouvertures étant détaillés dans la suite.

Le système comprend également en association avec chaque vis 10 un élément intermédiaire 30 et un écrou 40.

L'élément intermédiaire 30, qui présente une largeur qui est sensiblement égale à celle de la plaque 20, comporte un corps central 31 en forme générale de disque traversé par une ouverture centrale 37 d'un diamètre légèrement supérieur au diamètre hors-tout de la tige filetée 14 de la vis 10.

Dans deux régions diamétralement opposées du corps 31 s'étendent vers le bas deux jambes 32, 32' qui extérieurement épousent le contour circulaire du corps 31 et qui intérieurement présentent deux faces droites et parallèles 36, 36' situées en vis-à-vis, séparées par une distance légèrement supérieure à la distance entre deux pans opposés de la tête hexagonale 13 de la vis 10. Ces faces 36, 36' délimitent une face plane 39 constituée par la face inférieure du corps 31 de l'élément 30.

Ces jambes 32, 32' sont, à l'extrémité inférieure de leurs faces extérieures, légèrement arrondies pour éviter qu'elles définissent des arêtes vives susceptibles d'être traumatisantes.

Dans ces mêmes régions diamétralement opposées du corps 31 de l'élément 30 s'étendent vers le haut deux montants 33, 33' qui extérieurement épousent le contour circulaire du corps.

Ces montants présentent intérieurement des faces en biseau à 45°, respectivement 35 et 35', qui délimitent entre elles une zone de fond plane 34 constituée par la surface supérieure du corps 31.

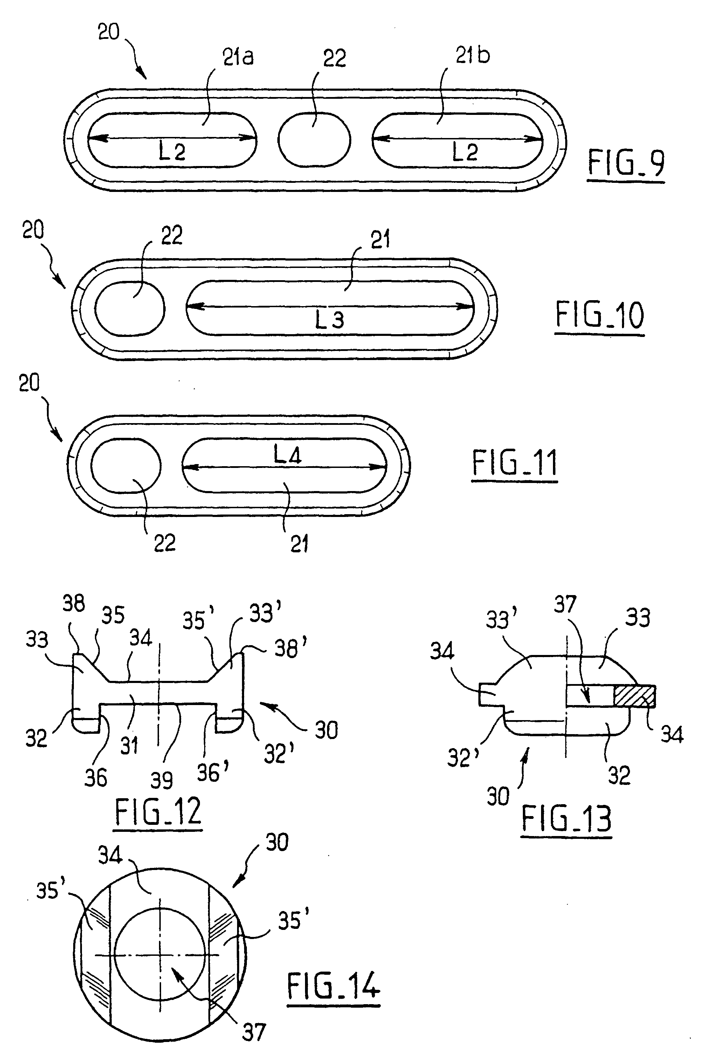

On observe en particulier sur la figure 12 que des méplats 38 parallèles à la face 34 assurent la transition entre les extrémités supérieures des faces 35, 35' et les extrémités supérieures des bords extérieurs des montants, ceci afin d'éviter la présence d'une arête très vive traumatisante.

Il est important d'observer ici que la largeur du fond 34 de l'élément est légèrement inférieure à la largeur entre chanfreins de la face inférieure 27 de la plaque 20, à des fins expliquées plus loin.

Enfin l'écrou 40 est du type classique en soi. Il comporte un corps 41 à six pans hexagonaux pour son serrage, surmonté d'une partie bombée 42 séparée dudit corps 41 par une fente transversale 43.

Le corps 41 et la partie bombée 42 sont traversées toutes deux par un taraudage axial, une légère déformation plastique de l'écrou par pincement au niveau de la fente 43 permettant de légèrement désaxer les deux parties du taraudage situées de part et d'autre de la fente 43, pour donner à l'écrou un caractère autobloquant.

L'implant tel que décrit ci-dessus se pose de la façon suivante.

Plusieurs vis pédiculaires 10 étant mises en place dans des vertèbres adjacentes, chaque vis commence par recevoir un élément 30, positionné de telle sorte que la tête 13 de la vis se trouve encastrée dans le canal défini, au-dessous de l'élément 30, par ses faces 36, 36' et 39, et de telle sorte que le sommet de ladite tête 13 soit en appui contre ladite face 39.

Les éléments 30 sont en outre orientés de telle sorte que les axes de leurs canaux supérieurs, définis entre leurs faces 34, 35 et 35', soient essentiellement alignés.

Une plaque 20 de géométrie appropriée est alors mise en place de telle sorte que les tiges filetées 14 des vis s'engagent dans des ouvertures traversantes 21 ou 22 de la plaque, et que la base de ladite plaque viennent s'appuyer dans le canal supérieur respectif de chaque élément, qui forme un berceau.

On observera ici que les faces à 45° 35 et 35' de chaque élément 30 assurent lors de cette opération, en coopération avec les parties latérales des chanfreins 26 de la plaque, un auto-centrage de la plaque sur l'élément respectif 30.

Les écrous 40 sont alors vissés sur les tiges filetées 14 des vis 10, pour amener la plaque 20 en compression dans son berceau respectif.

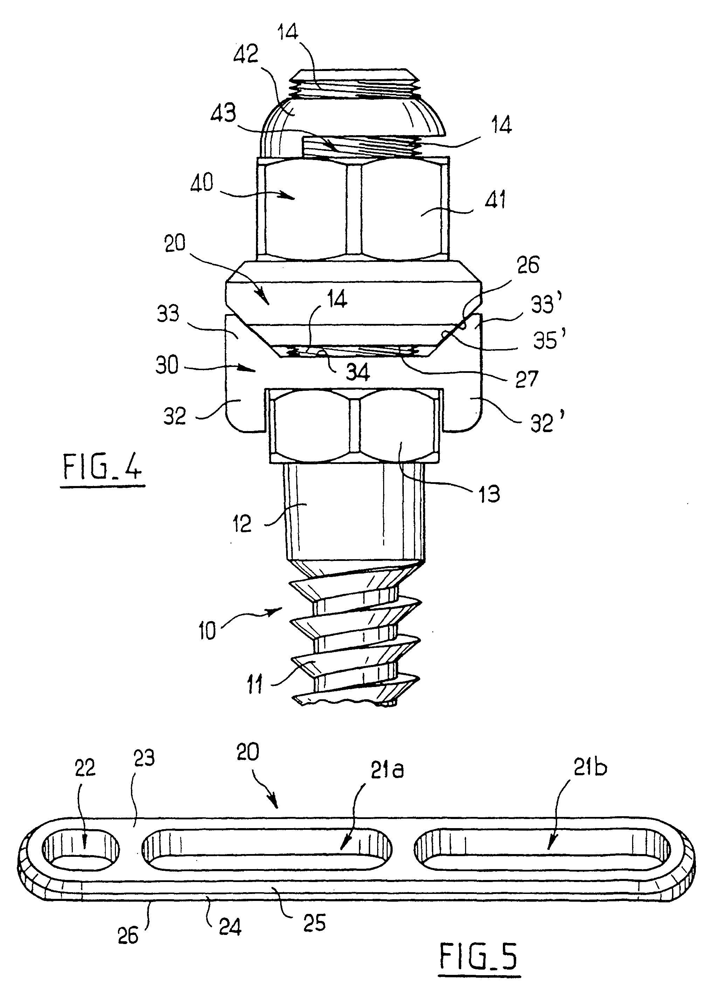

Il est à noter ici que, de façon particulièrement avantageuse, la largeur de la face inférieure 27 de la plaque étant plus grande que la largeur du fond 34 du berceau, la plaque 20 n'est en contact avec l'élément 30 qu'au niveau des faces à 45°, à savoir les chanfreins 26 et les faces en biseau 35, 35', et ceci même après serrage complet de l'écrou. Ainsi les figures 2 et 4 en particulier montrent qu'il subsiste après blocage un faible interstice, typiquement compris entre 0,5 et 2 mm, entre les faces respectives 27 et 34 de la plaque 20 et de l'élément 30.

Cette caractéristique a pour effet d'assurer une coopération entre la plaque 20 et chaque élément 30 selon des lignes de force obliques, qui induisent une légère déformation élastique des matériaux au niveau des faces en appui et donc des efforts de réaction importants.

Dans ces conditions, le frottement entre les faces en appui est suffisant pour pouvoir assurer un blocage parfaitement stable de l'ensemble et éviter le recours à des reliefs, crans ou autres destinés à éviter les glissements intempestifs.

On notera par ailleurs que, les élément 30 se trouvant, après la mise en place de la plaque 20, emprisonnés entre celle-ci et les têtes respectives 13 des vis 10, la technique opératoire est facilitée pour le chirurgien.

On va maintenant décrire certaines autres caractéristiques de l'invention, concernant en particulier la plaque 20.

Ainsi les figures 5, 8, 9, 10 et 11 montrent différents agencements des trous oblongs 21 et 22.

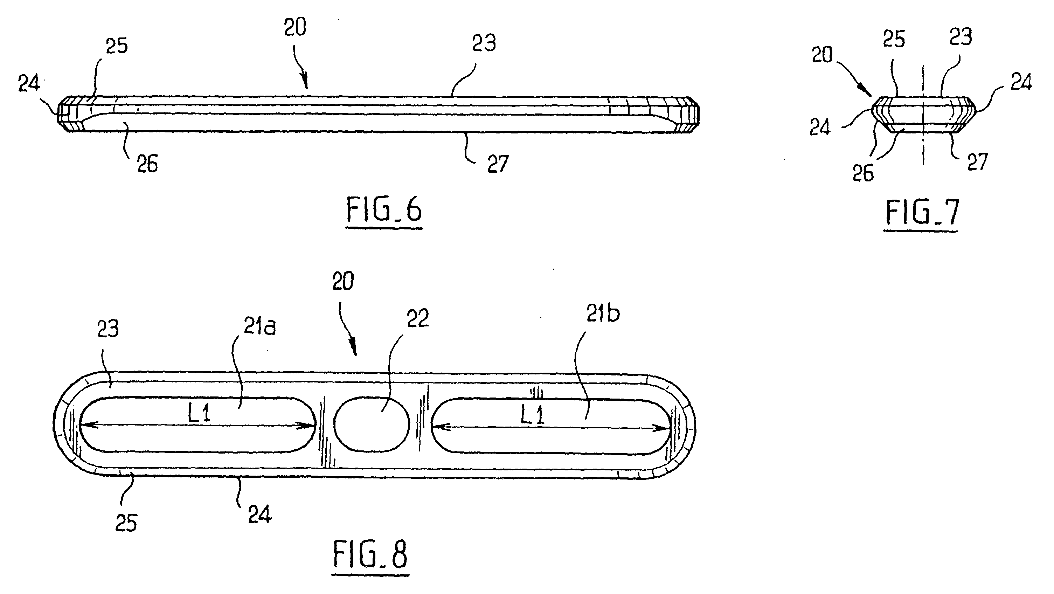

Dans le cas des figures 8 et 9, on trouve un trou court 22 entre deux trous longs 21, dont on peut proposer des versions avec différentes longueurs de trous longs (L1 sur la figure 8, et L2, inférieure à L1, sur la figure 9).

On peut également prévoir que le trou court 22 se trouve à une extrémité de la plaque, en association avec un trou long 21 (figures 10 et 11) ou deux trous longs 21a, 21b (figure 5).

Cette disposition permet en particulier, en disposant une plaque avec un trou court 22 au niveau d'une extrémité de la zone à traiter de la colonne vertébrale, d'éviter que le système d'implant déborde de façon indésirable vers la zone saine adjacente.

Dans le cas des figures 10 et 11, on a prévu deux trous longs 21 de différentes longueurs, respectivement L3 et L4.

Dans la pratique, on proposera au chirurgien un jeu de plaques, présentant toutes la même section transversale, ayant les différents agencements précités, ou d'autres agencements encore.

Une autre observation à propos des plaques est que celles-ci peuvent être dimensionnées de manière à présenter, entre deux vis pédiculaires, une certaine déformabilité élastique, qui permet d'améliorer la répartition des contraintes au niveau des différentes vertèbres.

Typiquement, lorsque les plaques sont réalisées dans un alliage de titane bio-compatible classique, on donne aux branches de la plaque bordant chaque trou 21 ou 22, outre leur forme particulière telle que décrite plus haut, une section transversale préférentiellement comprise entre 6 et 11 mm², et plus préférentiellement comprise entre 7,5 et 9,5 mm².

Bien entendu, la présente invention n'est nullement limitée aux formes de réalisation décrites ci-dessus et représentées sur les dessins, mais l'homme du métier saura y apporter toute variante ou modification conforme à son esprit.

## Revendications

1. Système d'ostéosynthèse pour le rachis, comprenant au moins deux vis pédiculaires (10) et une plaque d'union (20) apte à relier les vis l'une à l'autre de façon essentiellement rigide, chaque vis possédant une partie filetée (11) d'ancrage osseux et une tête (13) ayant une zone de section non circulaire, ainsi qu'une tige filetée terminale (14) apte à coopérer avec un écrou (40), la plaque possédant au moins une ouverture (21, 22) apte à être traversée par la tige filetée terminale d'une des vis pour être emprisonnée entre ladite tête et ledit écrou, et des moyens (30) étant prévus pour réaliser un blocage angulaire entre la tête (13) de chaque vis pédiculaire et la plaque, **caractérisé en ce que** les moyens de blocage angulaire comprennent un élément (30) rapporté entre la plaque et la tête de la vis pédiculaire et traversé par la tige filetée terminale de cette dernière, cet élément réalisant une première coopération de formes bloquante avec la tête (13) et une seconde coopération de formes bloquante avec la plaque (20), l'élément étant apte à réaliser la première coopération avec la zone de section non circulaire de la tête.

2. Système selon la revendication 1, **caractérisé en ce que** la première coopération de formes bloquante est un chevauchement de ladite tête à section non circulaire (13) par ledit élément rapporté (30) par le dessus.

3. Système selon la revendication 2, **caractérisé en ce que** ledit élément rapporté comprend deux jambes latérales (32, 32') délimitant entre elles un canal à bords droits dont la largeur est légèrement supérieure à la distance entre deux pans parallèles de ladite tête (13) de vis pédiculaire.

4. Système selon l'une des revendications 1 à 3, **caractérisé en ce que** la seconde coopération de formes bloquante est un chevauchement de ladite plaque (20) par ledit élément rapporté (30) par le dessous.

5. Système selon la revendication 4, **caractérisé en ce que** ledit élément rapporté possède deux montants latéraux (33, 33') définissant entre eux un canal à bords (35, 35') relevés en oblique, et **en ce que** ladite plaque (20) possède des chanfreins latéraux (26) aptes à s'appuyer contre lesdits bords obliques.

6. Système selon la revendication 5, **caractérisé en ce que** ladite plaque (20) s'appuie sur ledit élément rapporté (30) seulement au niveau desdits chanfreins (26).

7. Système selon l'une des revendications 1 à 6, **caractérisé en ce que** ledit élément rapporté (30) comporte une âme (31) en forme générale de disque traversée par une ouverture centrale (37) pour le passage de la tige filetée terminale (14) de la vis pédiculaire (10).

8. Système selon l'une des revendications 1 à 7, **caractérisé en ce que** la largeur de l'élément rapporté (30) est sensiblement égale à celle de la plaque (20).

9. Système selon l'une des revendications 1 à 8, **caractérisé en ce qu'**il comprend un jeu de vis pédiculaires (10) et un jeu de plaques (20) possédant différentes configurations de trous, avec un trou légèrement oblong (22) de géométrie constante et un ou plusieurs trous allongés (21; 21a, 21b) de longueurs différentes.

10. Système selon l'une des revendications 1 à 9, **caractérisé en ce que** l'écrou (40) vient en appui directement contre la face supérieure de la plaque (20).

## Patentansprüche

1. Osteosynthesesystem für die Wirbelsäule mit wenigstens zwei Fußschrauben (10) und einer Verbindungsplatte (20), die geeignet ist, die Schrauben in im wesentlichen steifer Weise miteinander zu verbinden, wobei jede Schraube einen gewindeten Bereich (11) zur Knochenverankerung und einen Kopf (13) mit einem nicht kreisförmigen Querschnittsbereich sowie eine gewindete Endstange (14) aufweist, die in der Lage ist, mit einer Mutter (40) zusammenzuwirken, wobei die Platte wenigstens eine Öffnung (21, 22) aufweist, welche dafür geeignet ist, von der gewindeten Endstange einer der Schrauben durchquert zu werden, um zwischen dem Kopf und der Mutter eingeschlossen zu werden, und wobei Vorrichtungen (30) vorgesehen sind, um eine Winkeiblockierung zwischen dem Kopf (13) jeder Fußschraube und der Platte herzustellen, **dadurch gekennzeichnet, dass** die Vorrichtungen zur Winkelblockierung ein Element (30) aufweisen, welches zwischen der Platte und dem Kopf der Fußschraube aufgesetzt ist, und welches von der gewindeten Endstange dieser letzteren durchquert ist, wobei dieses Element ein erstes blockierendes Formzusammenwirken mit dem Kopf (13) und ein zweites blockierendes Formzusammenwirken mit der Platte (20) bewirkt, wobei das Element in der Lage ist, das erste Zusammenwirken mit dem nicht kreisförmigen Querschnittsbereich des Kopfes herzustellen.

2. System gemäß Anspruch 1, **dadurch gekennzeichnet, dass** das erste blockierende Formzusammenwirken aus einer Überdeckung des Kopfes mit nicht kreisförmigem Querschnitt (13) durch das aufgesetzte Element (30) von oben her besteht.

3. System gemäß Anspruch 2, **dadurch gekennzeichnet, dass** das aufgesetzte Element zwei Seitenstreben (32, 32') aufweist, welche zwischen sich einen Kanal mit geraden Kanten begrenzen, dessen Breite etwas größer als der Abstand zwischen zwei parallelen Flächen des Kopfes (13) der Fußschraube ist.

4. System gemäß einem der Ansprüche 1 bis 3, **dadurch gekennzeichnet, dass** das zweite blockierende Formzusammenwirken aus einer Überdeckung der Platte (20) durch das aufgesetzte Element (30) von unten her besteht.

5. System gemäß Anspruch 4, **dadurch gekennzeichnet, dass** das aufgesetzte Element zwei seitliche Stützen (33, 33') aufweist, welche zwischen sich einen Kanal mit schräg hochgerichteten Kanten (35, 35') definieren, und dass die Platte (20) Seitenfasen (26) aufweist, welche in der Lage sind, sich gegen die schrägen Kanten zu stützen.

6. System gemäß Anspruch 5, **dadurch gekennzeichnet, dass** sich die Platte (20) nur auf der Höhe der Seitenfasen (26) auf das aufgesetzte Element (30) auflegt.

7. System gemäß einem der Ansprüche 1 bis 6, **dadurch gekennzeichnet, dass** das aufgesetzte Element (30) einen Kern (31) mit allgemeiner Scheibenform aufweist, der von einer Mittelöffnung (37) für den Durchgang der gewindeten Endstange (14) der Fußschraube (10) durchquert ist.

8. System gemäß einem der Ansprüche 1 bis 7, **dadurch gekennzeichnet, dass** die Breite des aufgesetzten Elementes (30) praktisch gleich derjenigen der Platte (20) ist.

9. System gemäß einem der Ansprüche 1 bis 8, **dadurch gekennzeichnet, dass** es einen Satz Fußschrauben (10) und einen Satz Platten (20) aufweist, welche verschiedene Lochkonfigurationen mit einem leicht länglichen Loch (22) mit konstanter Geometrie und einem oder mehreren langgestreckten Löchern (21; 21a, 21b) mit verschiedenen Längen aufweisen.

10. System gemäß einem der Ansprüche 1 bis 9, **dadurch gekennzeichnet, dass** die Mutter (40) direkt in Auflage gegen die Oberfläche der Platte (20) kommt.

## Claims

1. System for osteosynthesis of the spine, including at least two pedicular screws (10) and a connecting plate (20) adapted to connect the screws together in an essentially rigid manner, each screw having a bone anchor threaded part (11) and a head (13) having a part with a non-circular section, as well as a threaded end shank (14) adapted to cooperate with a nut (40), the plate having at least one opening (21, 22) adapted to have the threaded end shank of one of the screws passed through it to be trapped between the said head and the said nut, locking means (30) being provided for preventing relative angular movement between the head (13) of each pedicular screw and the plate, **characterized in that** the angular locking means include an attached member (30) between the plate and the head of the pedicular screw and through which the threaded end shank of the latter passes, this member providing a first locking cooperation of shapes with the head (13) and a second locking cooperation of shapes with the plate (20), the member being adapted to provide the first cooperation with the part of non-circular section of the head.

2. System according to Claim 1, **characterized in that** the first locking cooperation of shapes is a straddling of the said non-circular section head (13) by the said attached member (30) from above.

3. System according to Claim 2, **characterized in that** the said attached member has two lateral legs (32, 32') delimiting between them a channel with straight edges whose width is slightly greater than the distance between two parallel flats of the said head (13) of the pedicular screw.

4. System according to one of Claims 1 to 3, **characterized in that** the second locking cooperation of shapes is a straddling of the said plate (20) by the said attached member (30) from below.

5. System according to Claim 4, **characterized in that** the said attached member has two lateral columns (33, 33') defining between them a channel with oblique raised edges (35, 35') and **in that** the said plate (20) has lateral bevels (26) adapted to bear against the said oblique edges.

6. System according to Claim 5, **characterized in that** the said plate (20) bears only on the said bevels (26) of the said attached member (30).

7. System according to one of Claims 1 to 6, **characterized in that** the said attached member (30) has a core (31) which is generally disk-shaped with a central hole (37) through it through which the threaded end shank (14) of the pedicular screw (10) can be passed. ,

8. System according to one of Claims 1 to 7, **characterized in that** the width of the attached member (30) is substantially equal to that of the plate (20).

9. System according to one of Claims 1 to 8, **characterized in that** it includes a set of pedicular screws (10) and a set of plates (20) having different configurations of holes, with one slightly oblong hole (22) of constant geometry and one or more elongate holes (21; 21a, 21b) of different lengths.

10. System according to one of Claims 1 to 9, **characterized in that** the nut (40) bears directly against the top face of the plate (20).
